(19)

Europäisches Patentamt

European Patent Office

Office européen des brevets

(11) **EP 2 777 658 A1**

(12) **EUROPEAN PATENT APPLICATION**

(43) Date of publication:
**17.09.2014 Bulletin 2014/38**

(51) Int Cl.:
*A61F 5/56* (2006.01)

(21) Application number: **14160389.4**

(22) Date of filing: **17.03.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **15.03.2013 US 201361799129 P**

(71) Applicants:
• **PX3 Sports Science**
  **Calgary, AB T3M 1A9 (CA)**
• **P3 Athletics Inc. trading as PX3 Sports Science**
  **Calgary, AB T3M 1A9 (CA)**

(72) Inventor: **Layzell, P. Bradley**
  **Calgary, Alberta T3M-1A9 (CA)**

(74) Representative: **Bates, Daniel Joseph**
  **Ogive Intellectual Property Ltd**
  **34 Westgate**
  **North Cave**
  **Brough HU15 2NJ (GB)**

(54) **Performance enhancing bite regulator and method of custom fitting same**

(57) A bite regulator has a front band with left and right ends and left and right molar sections extending rearward respectively from the left and right ends of the front band. The left and right molar sections each have an interocclusal region with a contoured top surface and a contoured bottom surface. The contoured top and bottom surfaces are each configured to match a specific contour of the wearer's top and bottom teeth, respectively, and are aligned relative to one another to correctly position the wearer's jaw.

FIG. 1

EP 2 777 658 A1

## Description

### Related Application Data

This patent is related to and claims priority benefit of U.S. provisional application Serial No. 61/799,129 filed March 15, 2013 and entitled "Performance Enhancing Bite Regulator and Method of Custom Fitting Same." The entire content of this prior filed provisional application is hereby incorporated herein by reference.

### Background

#### 1. Field of the Disclosure

The present invention relates generally to oral appliances and products, and more particularly to a bite regulator that regulates a wearer's jaw and bite position during use to increase oxygen, neurological, and physiological response and maintain proper neuromusculoskeletal alignment under high impact circumstances and in high stress environments.

#### 2. Description of Related Art

There are many appliances and products that are to be worn in the mouth. Such appliances also can serve many different purposes. Some of these products are intended to be worn while participating in athletic endeavors. Many appliances of this type are worn by athletes as mouth guards and are intended to protect the athlete's teeth.

U.S. Patent No. 7,404,403 discloses an oral appliance in the form of a mouth guard or sports guard. The sports guard 1 is to be placed in a mouth of a user. The guard 1 has a base member 2 with a generally U-shaped form corresponding to the outline of a jaw of a user and defines an upper channel 10 within which the upper row of teeth of a user are received. The guard also has a teeth-engaging element 3 associated with the channel 10 and made of a material that can be shaped or formed by a user to be molded to their mouth. The guard also has a shock absorption aspect associated with the base member 2 for absorbing impact shock. The shock absorption aspect utilizes open channels in the base member 2 that are to simulate air springs. The base member also has a polyethylene mixed with up to 10% of EVA. The addition of EVA is said to give the guard more flexibility. The teeth engaging element is made of EVA.

U.S. Patent No. 6,082,363 (E-Z Gard Industries, Inc.) discloses a triple layer mouth guard with an integral shock absorbing framework. The triple layer mouth guard apparatus has a U-shaped mouth guard base with an elastomeric frame embedded therein. The frame includes a wave-shaped contact surface. The mouth guard base has inner and outer side walls that define an upper channel, which has a liner disposed therein. The liner is adapted to form around the teeth of the wearer when softened. The liner engages the teeth of the upper jaw and is made of a material that softens at a temperature lower than the material of the mouth guard base and remains softer than the material of the mouth guard base when hardened.

U.S. Patent No. 7,210,483 (Medtech Products, Inc.) discloses an interocclusal sports prophylaxis that includes a core having an arch-shaped occlusal plate with upwardly extending buccal and lingual walls. A labial force dispersal shield and molar framing braces extend downwardly from the plate at incisor and lingual zones of the plate. The occlusal plate is thickened at the molar and incisor zones so that breathing spaces are provided between mandibular occlusal surfaces not registered with the zones and a mandibular face of the plate. A shock absorption dentition encasement is molded over the upper surface of the core and also covers the molar and incisor zones. The prophylaxis is heated in water and then seated in the mouth. Biting pressure is applied to embed the maxillary dentition in the encasement and the lower incisor and molar occlusal surfaces in the encasement covering the zones.

U.S. Patent No 7,305,990 (Den-Mat Corporation) discloses a mouth guard and kit with a user-configured mouth guard. The user's teeth and gums are not directly exposed to the uncured mouth guard material. The uncured mouth guard material is pliant at room temperature and does not have to be heated to form and shape the mouth guard. The cured mouth guard can be pliant or rigid at room temperature. A kit and a method of forming the mouth guard are also disclosed.

U.S. Patent No. 6,830,051 (Dental Concepts LLC) discloses an interocclusal appliance that includes a maxillary impression preform of a resilient thermoplastic having a low softening temperature, e.g. 36 degree C, such as an EVA copolymer having approximately thirty percent vinyl acetate. The preform is molded over and unitarily bonded to a base having a planar bottom face contacted by mandibular occlusal surfaces. The base is formed of a thermoplastic having a higher softening temperature, e.g. 70 degree C, with the bond between the preform and base characterized by high shear strength. The preform includes a bight shaped centric relation positioning channel having a thick footing and draft along lingual, buccal, and labial walls. The appliance is fitted by immersion in hot water to soften the preform, seating the maxillary arch within the channel and biting, such that the impression of the maxillary dentition embeds in the softened preform. Upon cooling, the preform is transformed into a reusable resilient encasement for the maxillary dentition. Suitable thermoplastics for implementation as the base include an EMA copolymer, blends of EMA and EVA or TPU, or blends of TPU and EVA.

The foregoing products may offer some type of improvement over standard, inexpensive athletic mouth guards. However, all have significant disadvantages as well. Such disadvantages are discussed below throughout the detailed description but generally include not accounting for a user's natural jaw position, restricting the user's airway, restricting verbal communication, and the like.

## Summary

In one example according to the teachings of the present disclosure, a bite regulator has a front band with left and right ends and left and right molar sections extending rearward respectively from the left and right ends of the front band. The left and right molar sections each have an interocclusal region with a contoured top surface and a contoured bottom surface. The contoured top and bottom surfaces are each configured to match a specific contour of the wearer's top and bottom teeth, respectively, and are aligned relative to one another to correctly position the wearer's jaw.

In one example, the front band can be a labial band configured to contact only an outer facing side of a wearer's front teeth.

In one example, the front band can be a labial band that has a tooth facing inner surface that is contoured to match a front surface contour of the wearer's front teeth.

In one example, the bite regulator can be a lower bite regulator and a top edge of the front band can terminate at or below a height of a user's bottom front teeth.

In one example, the bite regulator can be a lower bite regulator and a bottom edge of the front band can be contoured to follow a soft tissue line between the gums and the wearer's front teeth but not overlie the soft tissue.

In one example, the left and right molar sections each can have an interocclusal region with a contoured top surface and a contoured bottom surface each configured to match a contour of a specific wearer's top and bottom teeth, respectively. The contoured top and bottom surfaces can be aligned relative to one another to correctly position the wearer's jaw.

In one example, the left and right molar section each can have a lingual wall on an inner side of the respective molar section. A tongue shelf can be formed on each of the lingual walls.

In one example, the bite regulator can be a lower bite regulator and can have a lingual wall that wall depends from a respective inner side of each molar section and that has a bottom edge that is contoured vertically to follow a contour of the soft tissue line of a wearer and does not contact or overlap any soft tissue below the molars of the wearer.

In one example, the left and right molar sections each can have a buccal wall on an outer side of the respective molar section.

In one example, the bite regulator can be a hybrid lower bite regulator and can have buccal wall that depends from a respective outer side of each molar section and can have a bottom edge positioned below the soft tissue line of a wearer so that the buccal wall contacts or overlaps the soft tissue below the lower molars of the wearer.

In one example, the bite regulator can be a hybrid lower bite regulator with the front band having a front wall portion that can at least partially cover a front side of a wearer's lower front teeth and a rear wall portion that can at least partially cover a back side of the wearer's lower front teeth.

In one example, the bite regulator can be an upper bite regulator with a V-shaped front band that can have a labial wall and a lingual wall connected to one another along a bottom edge. The lingual wall can have a top edge that is contoured vertically to follow a contour of the soft tissue line of a wearer and that does not contact or overlap any soft tissue above the front teeth of the wearer.

In one example according to the teachings of the present disclosure, a bite regulator has a front band with left and right ends and left and right molar sections extending rearward respectively from the left and right ends of the front band. The left and right molar sections terminate short of a fourth rearward molar of a user.

In one example, the bite regulator can have a rear lip protruding from a free end of each of the left and right molar sections. Each rear lip can be configured to lie between a third molar and the fourth molar of a user and against a rear edge of the third molar.

In one example, the bite regulator can be a lower bite regulator and can have a rear lip that depends downward from a respective free end of each of the corresponding molar sections.

In one example, the bite regulator can be an upper bite regulator and can have a rear lip that protrudes upward from a respective free end of each of the corresponding molar sections.

In one example according to the teachings of the present disclosure, a method of fitting a bite regulator includes providing one or more bite forks capable of positioning and holding a subject's jaw in a correct jaw position specific to the subject. An upper impression of the subject's upper teeth is prepared and a lower impression of the subject's lower teeth is prepared while the subject bites down on the bite fork in the correct jaw position. A bite regulator is then molded using the upper and lower impressions and maintaining the correct jaw position of the lower impression relative to the upper impression.

In one example, the method can include forming a front band between left and right molar sections of the bite regulator. Each molar section can be formed having a lingual wall with a free edge that is contoured vertically to follow a contour of the soft tissue line of the subject and so that the lingual wall does not contact or overlap any soft tissue adjacent the molars of the subject.

In one example, the method can include forming a front band between left and right molar sections of the bite regulator. The left and right molar sections can terminate short of a fourth rearward molar of the subject.

In one example, the method can include forming a front band between left and right molar sections of the bite regulator. The left and right molar sections can terminate short of a fourth rearward molar of the subject. A rear lip can be formed protruding from a free end of each of the left and right molar sections and can be configured to lie between a third molar and the fourth molar of the subject and against a rear edge of the third molar.

**Brief Description of the Drawings**

Objects, features, and advantages of the present invention will become apparent upon reading the following description in conjunction with the drawing figures, in which:

FIG. 1 shows a perspective view of one example of a lower bite regulator constructed in accordance with the teachings of the present disclosure.

FIG. 2 shows a front view of the lower bite regulator of FIG. 1.

FIG. 3 shows a top view of the lower bite regulator of FIG. 1.

FIG. 4 shows a bottom view of the lower bite regulator of FIG. 1.

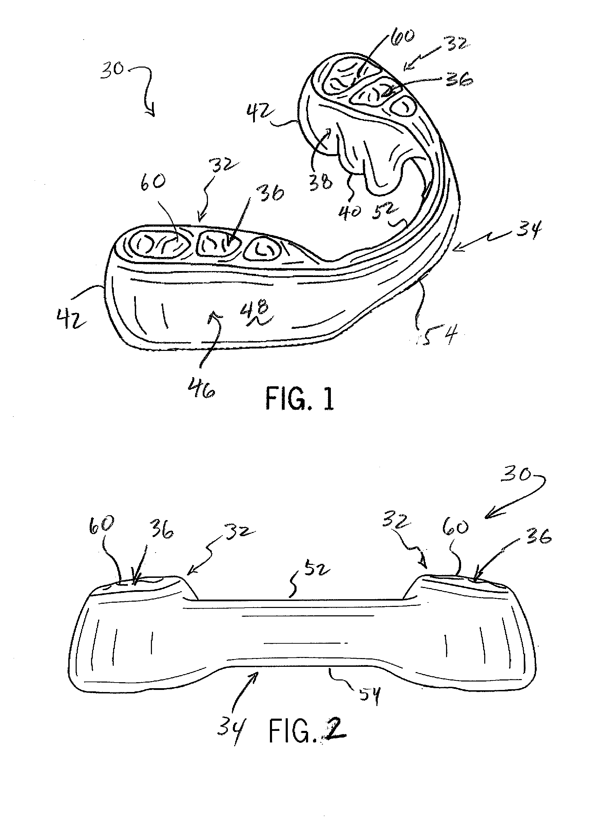

FIG, 5 shows a side view of the lower bite regulator of FIG. 1.

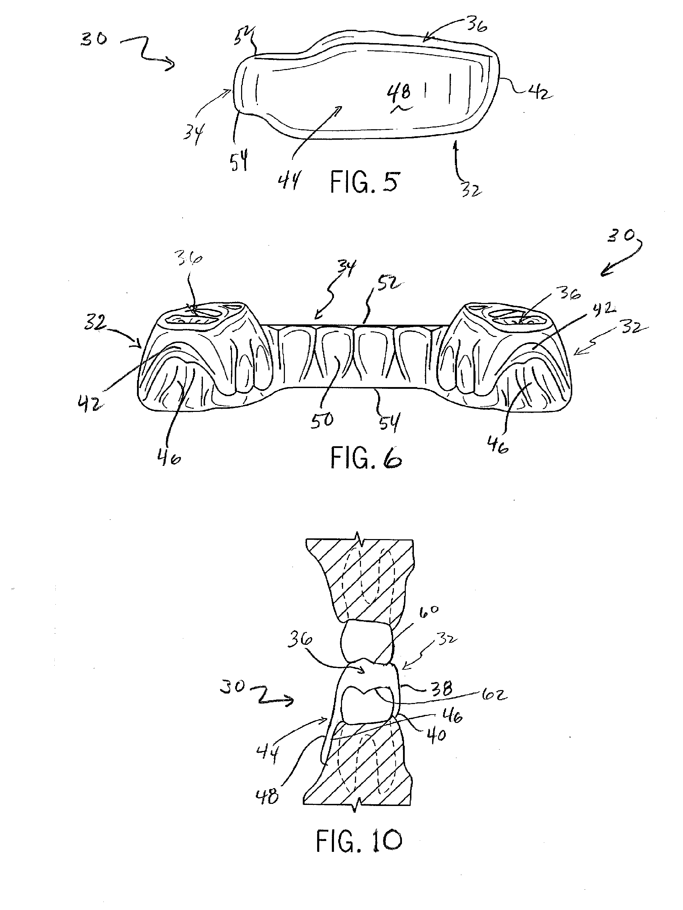

FIG. 6 shows a rear view of the lower bite regulator of FIG. 1.

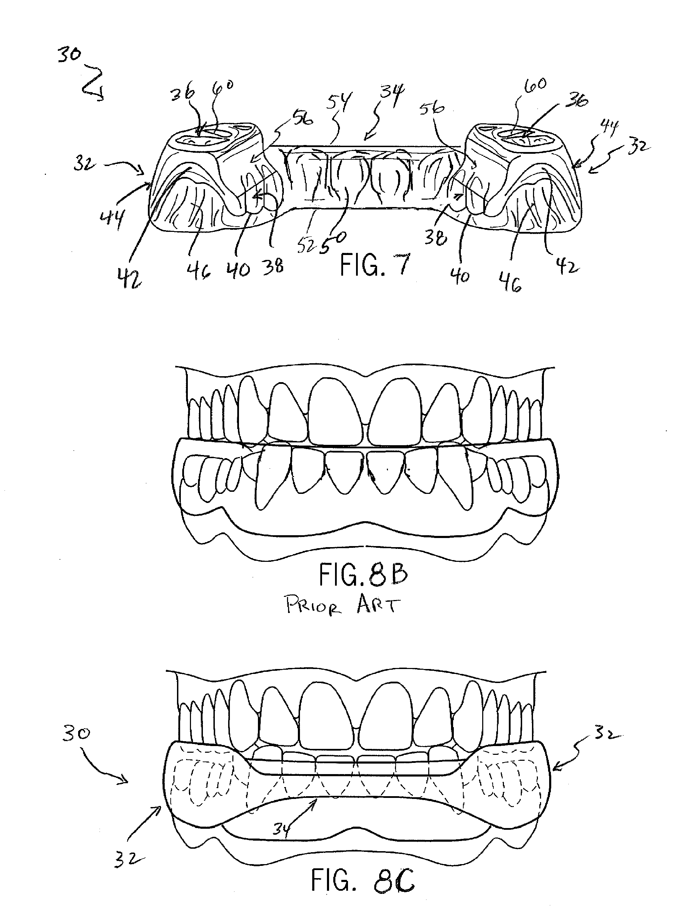

FIG. 7 shows a rear view of the lower bite regulator of FIG. 1, but with an optional tongue shelf on the lingual walls.

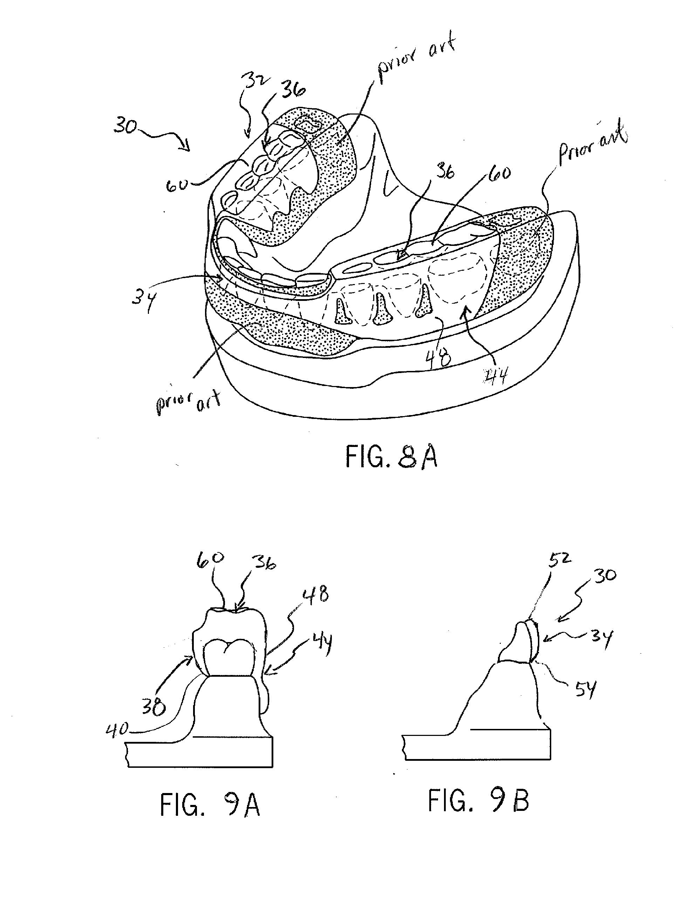

FIG. 8A shows a comparison between a prior art oral appliance and the lower bite regulator of FIG. 1 and on a lower set of teeth.

FIG. 8B shows a front view of a prior art oral appliance on a lower set of teeth.

FIG. 8C shows a front view of the lower bite regulator of FIGS. 1 and 8A on a lower set of teeth.

FIGS. 9A and 9B show cross section views of the lower bite regulator taken along lines 9A-9A and 9B-9B of FIG. 8A.

FIG. 10 shows a cross-section of the lower bite regulator between upper and lower molars of a wearer.

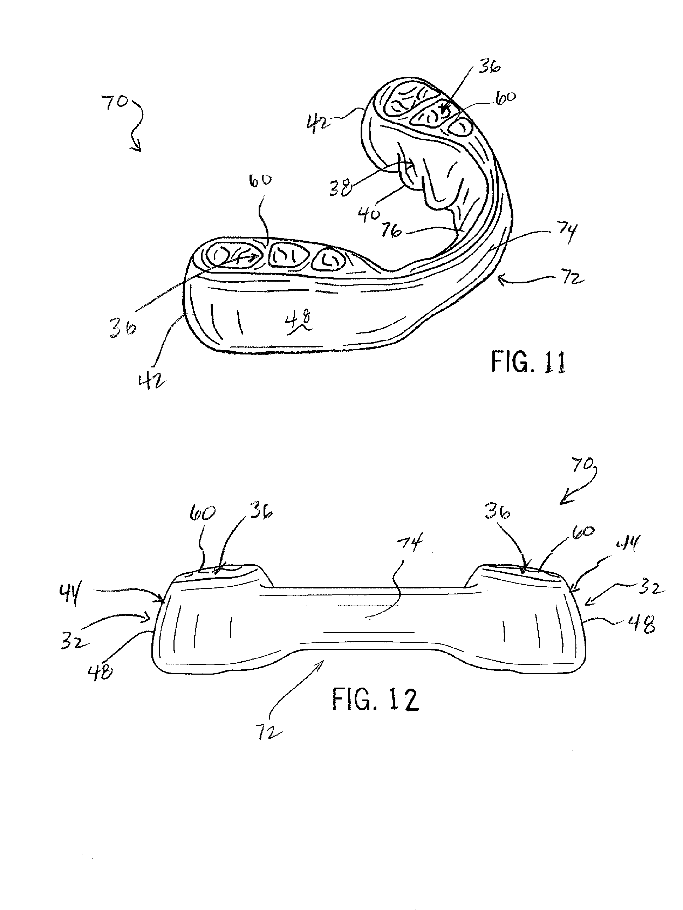

FIG. 11 shows a perspective view of one example of a hybrid lower bite regulator constructed in accordance with the teachings of the present disclosure.

FIG. 12 shows a front view of the lower bite regulator of FIG. 11.

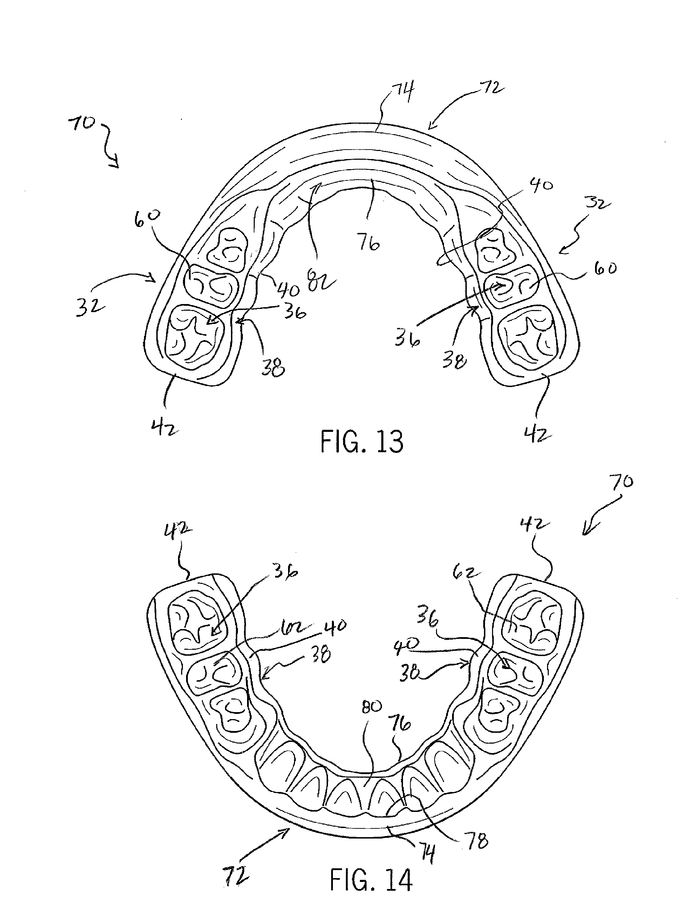

FIG. 13 shows a top view of the lower bite regulator of FIG. 11.

FIG. 14 shows a bottom view of the lower bite regulator of FIG. 11.

FIG, 15 shows a side view of the lower bite regulator of FIG. 11.

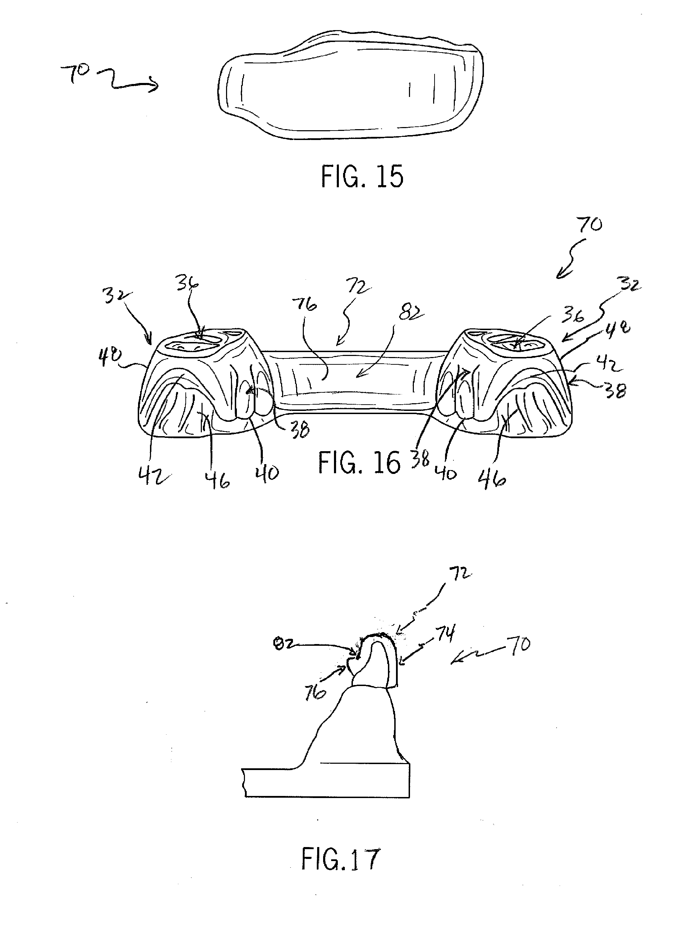

FIG. 16 shows a rear view of the lower bite regulator of FIG. 11.

FIG. 17 shows a cross section of the front band of the lower bite regulator of FIG. 1 and on a set of lower teeth.

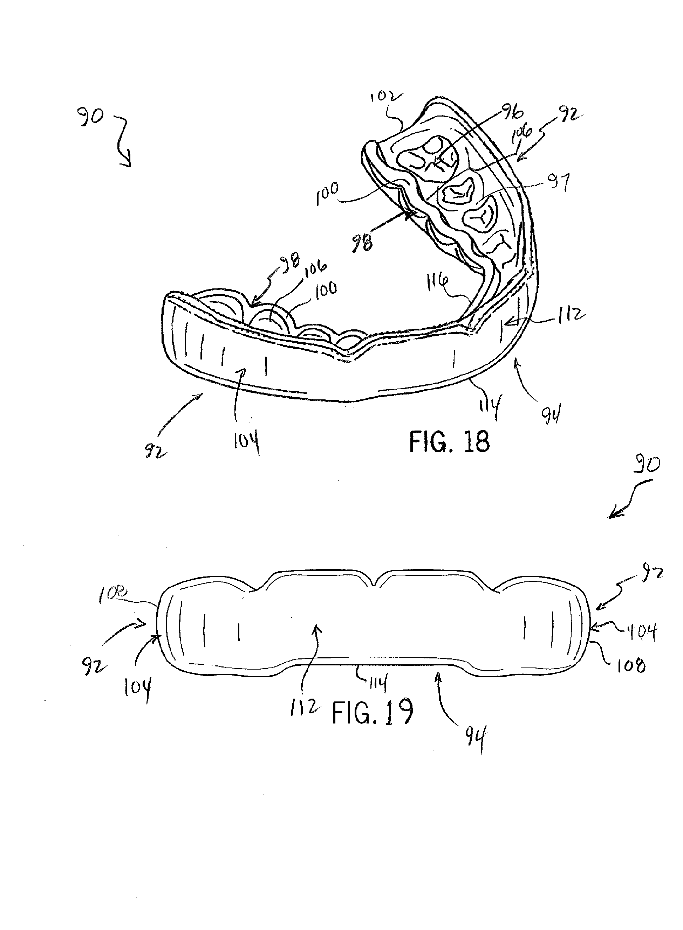

FIG. 18 shows a perspective view of one example of a hybrid lower bite regulator constructed in accordance with the teachings of the present disclosure.

FIG. 19 shows a front view of the lower bite regulator of FIG. 18.

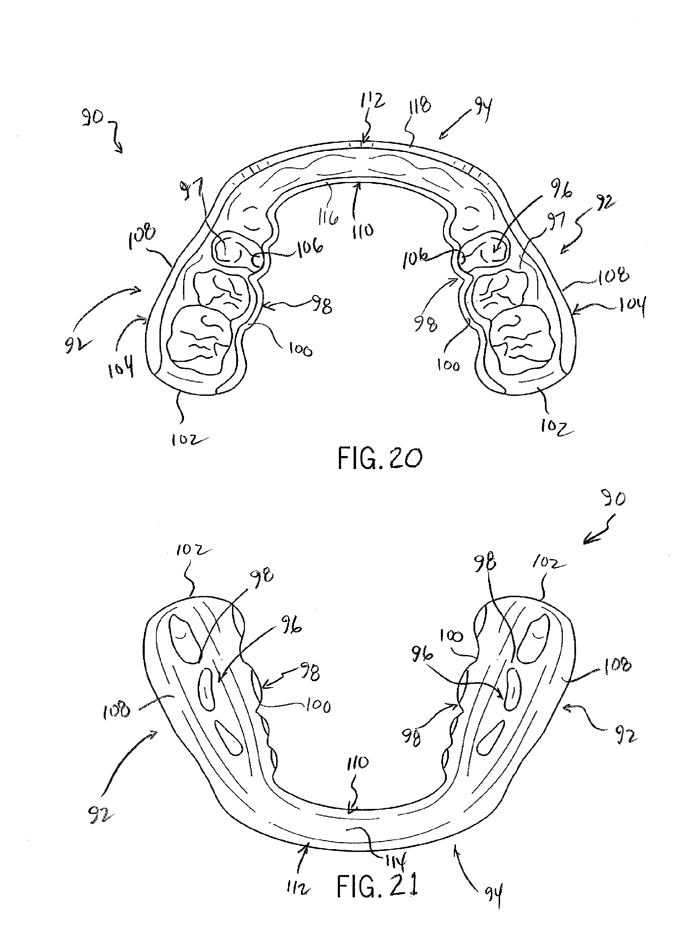

FIG. 20 shows a top view of the lower bite regulator of FIG. 18.

FIG. 21 shows a bottom view of the lower bite regulator of FIG. 18.

FIG, 22 shows a side view of the lower bite regulator of FIG. 18.

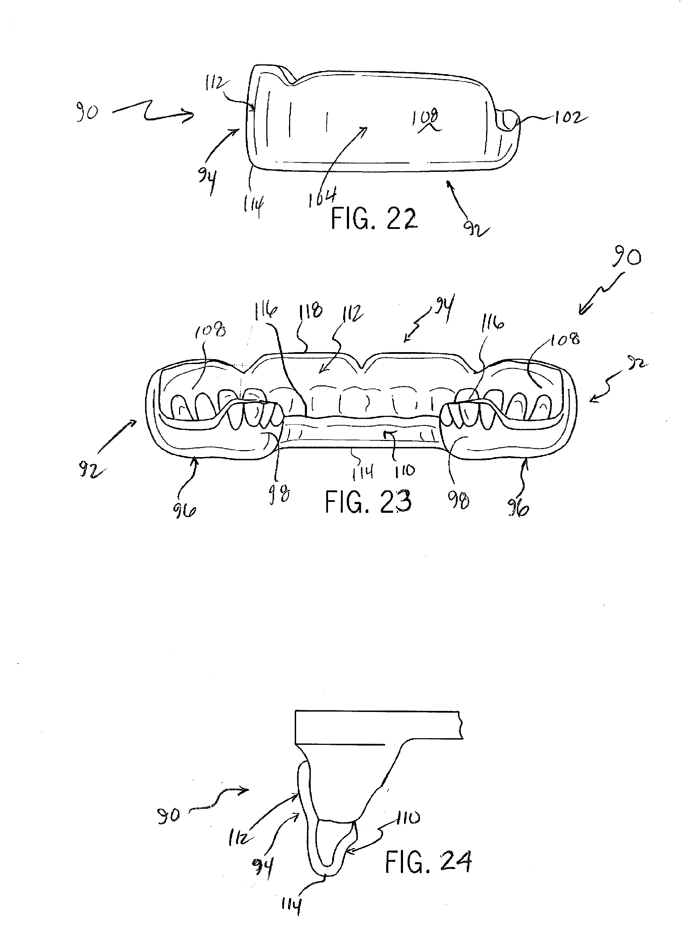

FIG. 23 shows a rear view of the lower bite regulator of FIG. 18.

FIG. 24 shows a cross section of the front band of the upper bite regulator of FIG. 18 and on a set of teeth.

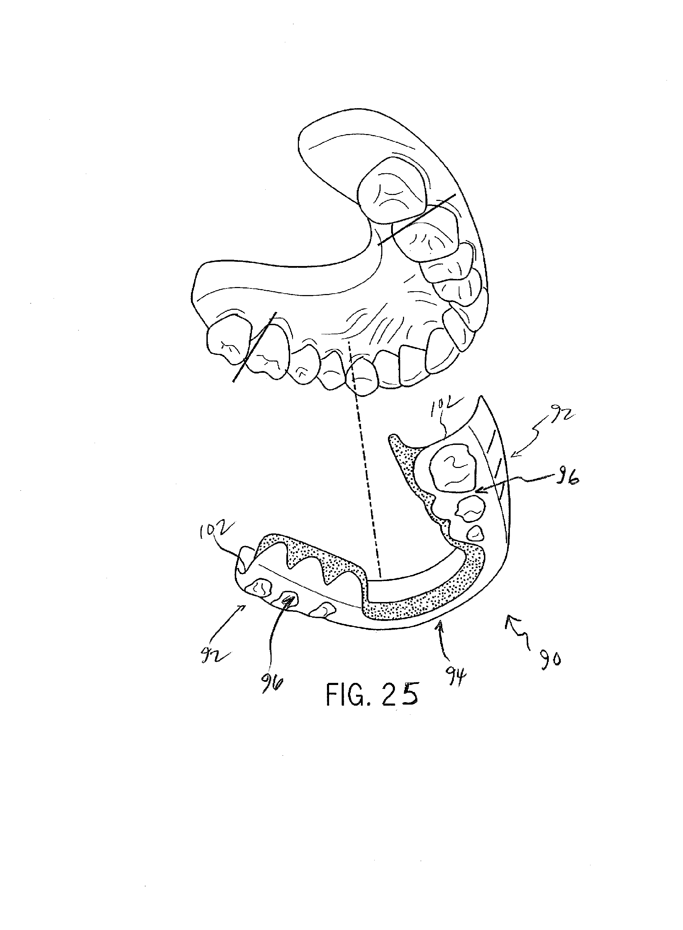

FIG. 25 shows a perspective view of the upper bite regulator of FIG. 18 spaced from a set of upper teeth and showing differences between a prior art mouth piece and the upper bite regulator.

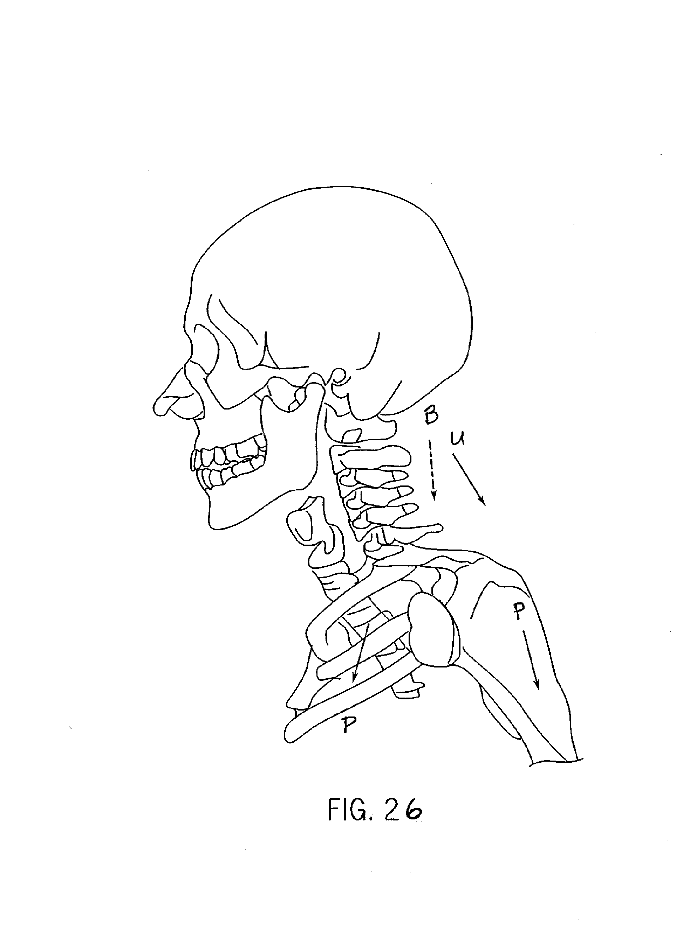

FIG. 26 shows a schematic representation of pull forces exerted by a person's head on the neck muscles.

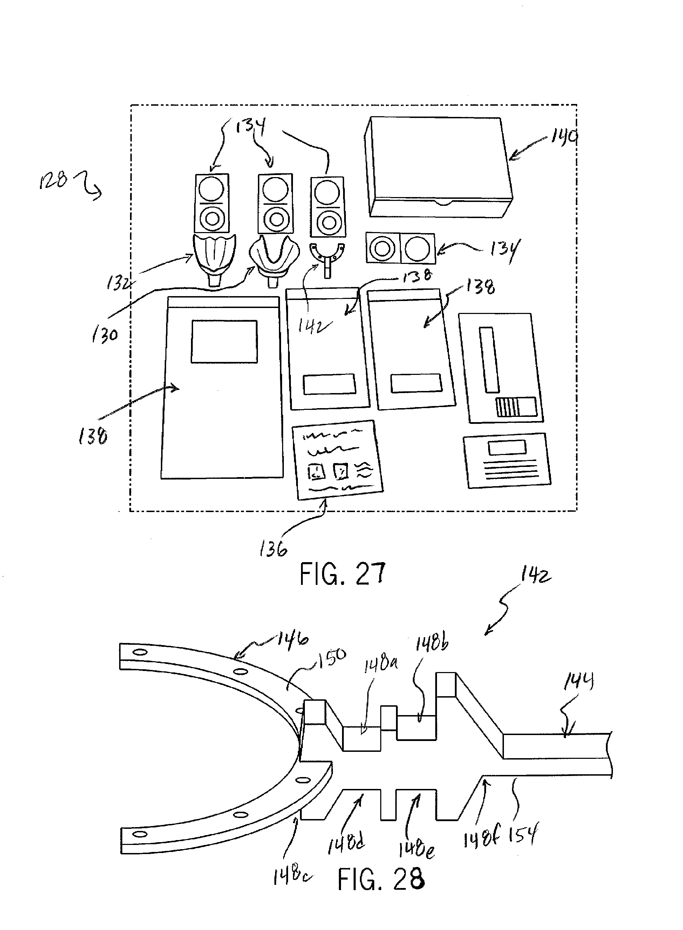

FIG. 27 shows one example of a self-impression kit for fitting a bite regulator according to the teachings of the present disclosure.

FIG. 28 shows one example of a bit fork for the self-impression kit of FIG. 27.

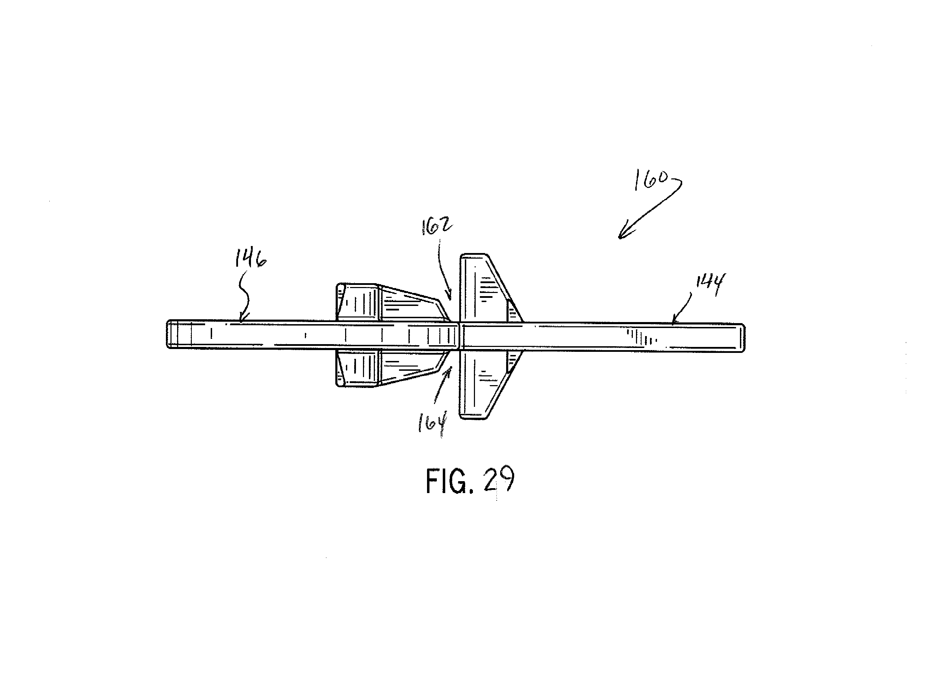

FIG. 29 shows another example of a bite fork for the self-impression kit of FIG. 27.

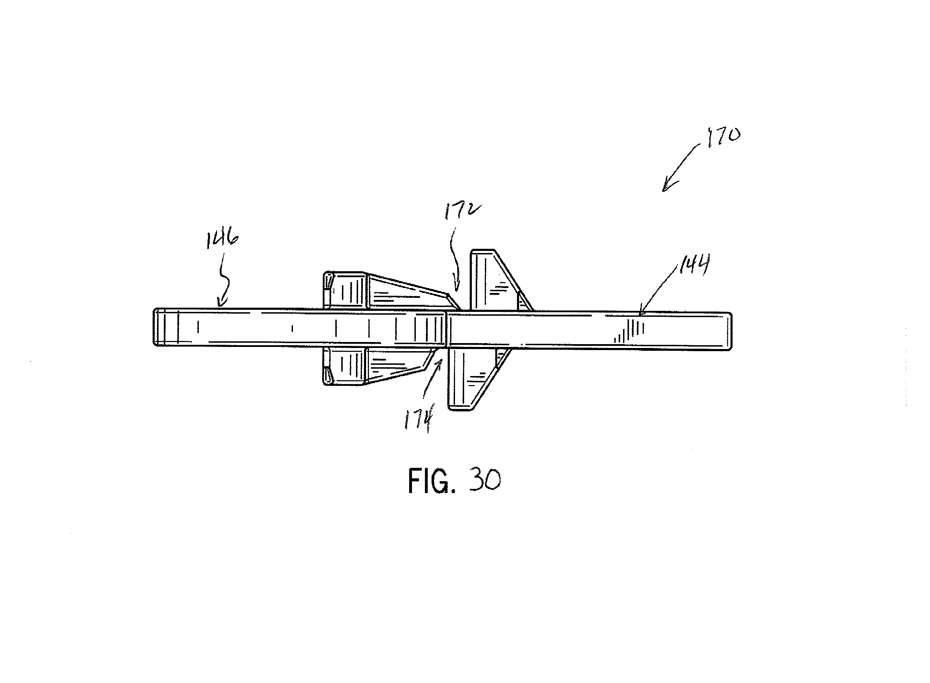

Fig. 30 shows another example of a bite fork for the self-impression kit of FIG. 27 and in a first orientation.

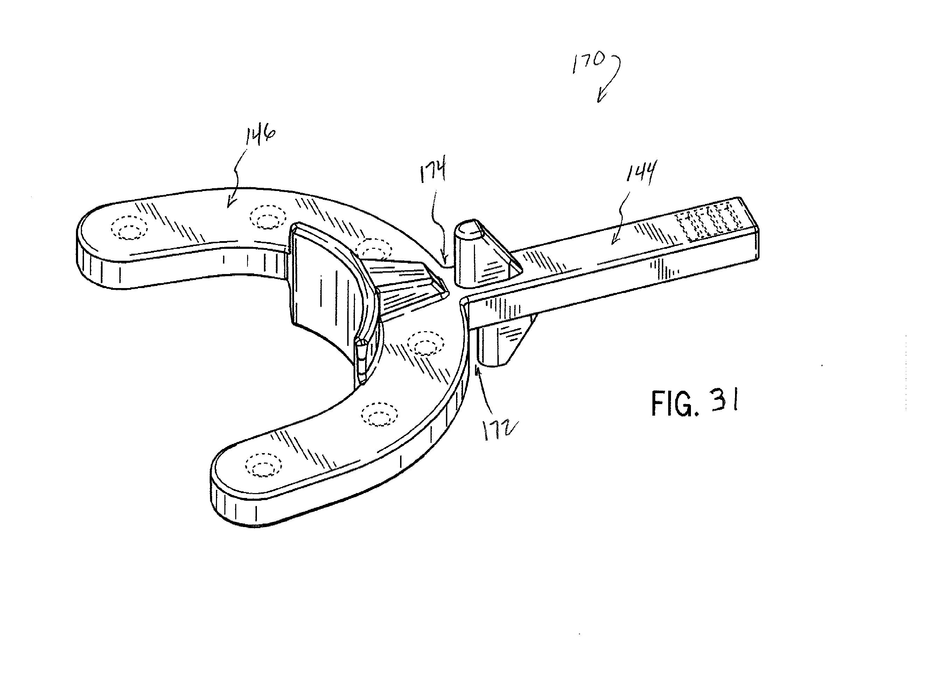

FIG. 31 shows the bite fork of FIG. 30 in a flipped or inverted orientation.

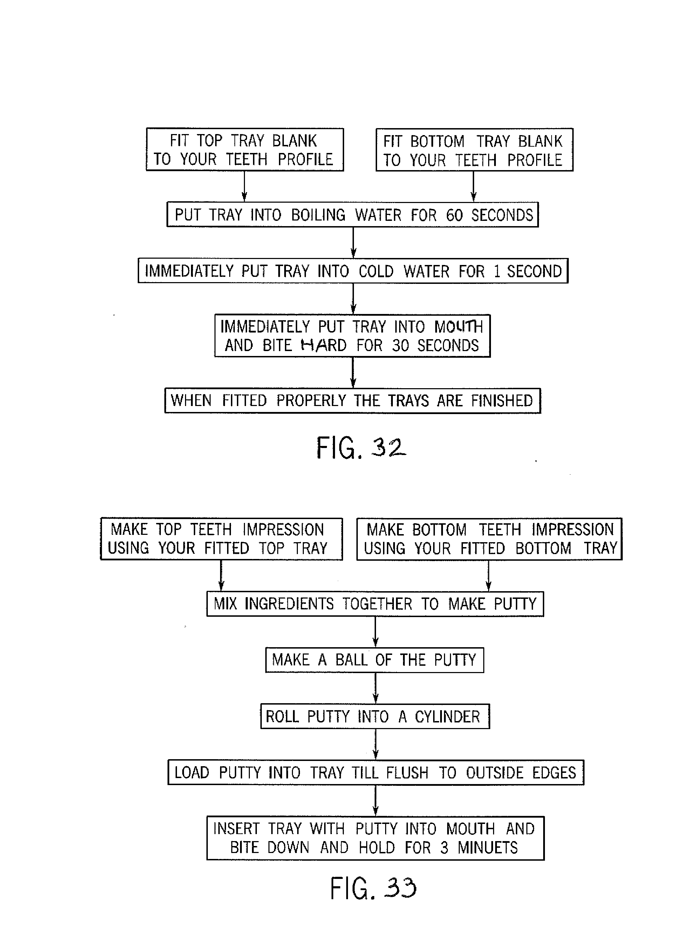

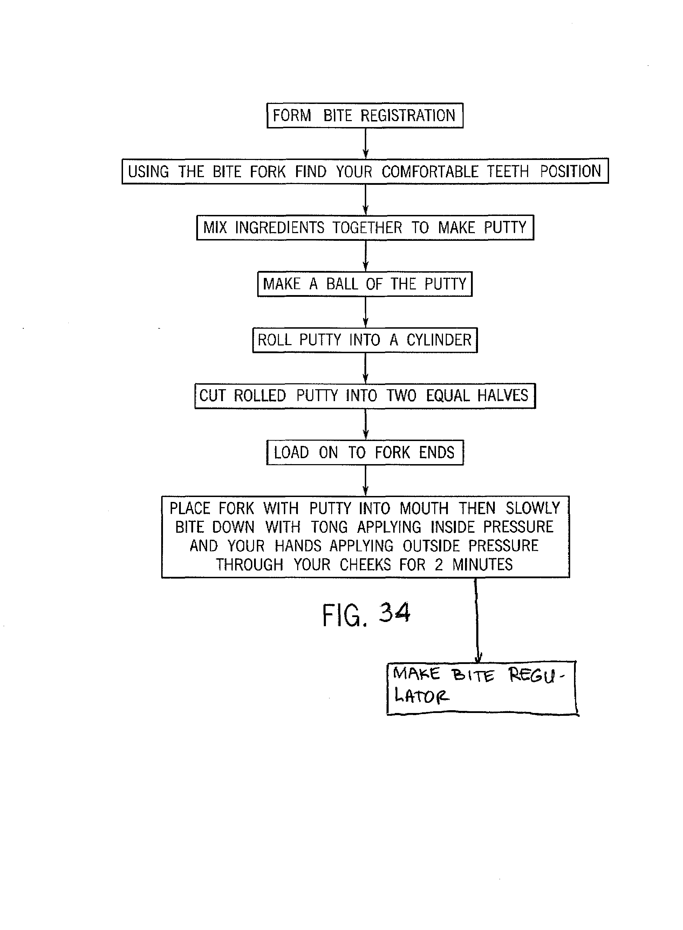

FIGS 32 -34 show one example of a detailed fitting process for utilizing the self-impression kit of FIG. 27.

**Detailed Description of the Disclosure**

The disclosed invention is for an oral appliance called the PX3 Bite Regulator. The product design solves or improves upon one or more problems and disadvantages with existing and prior known oral appliances, mouth guards, and the like.

Scientific research conducted using the disclosed oral appliances shows proven ground breaking improvements in product performance, and more important, in the performance of the athlete or user of the product. The research and test results have shown that the products can significantly reduce the occurrence of concussions in contact sports, sometimes by as much as 80% or more. The disclosed oral appliances may be a suitable or superior replacement for virtually every mouth guard on the market. The disclosed oral appliances is proven to improve performance of individuals as they undertake any number of activities, not just contact sports, whether for youth, high school, recreational, collegiate, professional, or Olympic competition. The disclosed oral appliances have also been proven to improve the performance of military personnel, firemen, members of law enforcement, first responders and the like.

The disclosed oral appliance can be categorized and described as fully customized bite-positioning mouthpieces that maintain optimized jaw alignment and allow for enhanced and unobstructed breathing. The disclosed mouthpieces also allow for unobstructed communication while the products are being used. The disclosed PX3 Custom Bite Regulators are scientifically proven to increase oxygen volumes, heart rate variability, cognitive processing and dopamine (neurotransmitters directly linked with movement, attention, and learning), and reduces stress, anxiety and promotes a more efficient operation of the autonomic nervous system in individuals using the product in comparison to using prior or no mouthpieces.

During high impact and high stress environments, the human body's natural response is to go into a defensive state, clench the jaw and stop breathing. Lack of oxygen shuts down parts of the brain that control fine motor skills, vision, hearing, depth perception that make simple tasks more difficult, resulting in poor performance, higher risk of mistakes and of injury. While using the disclosed PX3 Bite Regulators, testing has shown that an athlete is able to transfer higher oxygen levels by properly aligning and stabilizing the jaw position, thereby eliminating the ability to clench the teeth together and completely shut off the flow of oxygen. This feature allows the person to maintain a consistent flow of oxygen to the brain, remain calm and focused and to execute when normally they would not be able to. The effects of this increase in oxygen on training and cognitive function compounding day after day, week after week, and month after month, are measurably significant. For these types of high stress, high impact, and/or high intensity environments, it is simply not possible for one to perform at the same level with no mouthpiece, and even more difficult with a traditional mouthguard where breathing and communication is restricted through excess material or jaw misalignment.

Traditional mouth guards are not designed to increased breathing or stabilize the jaw, they are designed to for protecting teeth, lips and gums. Some protective headgear products, such as football helmets, are strapped tight to a wearer's head by a taught chinstrap. This can further misalign the player's jaw, making it even harder to breath, and communicate. This further increases stress on the autonomic nervous system, reduces their ability to function, both mentally and physically and puts the athlete at a much higher risk of getting hurt or in the case of military combat, getting killed or doing long-term permanent damage. The same can be said for many individuals that regularly must perform under stressful conditions.

The disclosed oral appliances, i.e., the Custom Bite Regulators promote and enhance advanced human performance. The products are fully natural, clean, safe, and can be useful for all levels and types of competition and physical activity. The disclosed Bite Regulators are not a corrective orthotic for severe jaw disorders or pain management. The Bite Regulators are specifically designed for increasing respiratory, physiological and neurological performance in high performance, high stress and high impact conditions. The disclosed Bite Regulators are not a mouth guard or gum shield. Custom mouth guards involve one (1) single impression of the wearer's teeth and are designed for full dental coverage and minor tooth, lip and gum protection. The disclosed Bite Regulators involve nine (9) unique custom measurements: 1) full anatomy of the maxillary teeth; 2) full anatomy of the mandibular teeth; 3) full labial registration of the upper or lower gums, depending on the mouth piece model; 4) precise left side vertical positioning of maxillary and mandibular lateral incisors, canine, premolar, and molars; 5) precise right side vertical positioning of maxillary and mandibular lateral incisors, canine, premolar, and molars; 6) precise left side horizontal positioning of maxillary and mandibular lateral incisors, canine, premolar, and molars; 7) precise right side horizontal positioning of maxillary and mandibular lateral incisors, canine, premolar, and molars; 8) precise vertical positioning of maxillary and mandibular centric incisors; and 9) precise horizontal positioning of maxillary and mandibular centric incisors.

Athletic Mouth guards and many custom orthotics restrict airway, communication, misalign the bite or do not stabilize the jaw, and indirectly increase the risk of injury by reducing individual performance levels during even minimal levels of physical activity. The disclosed Bite Regulators are fitted using custom impressions of top and bottom arches, jaw position, and proper vertical, sagittal and horizontal bite position. Existing mouthwear does not encompass all of these parameters. The disclosed Bite Regulators are designed to provide enhanced postural alignment by aligning the jaw, unobstructed breathing, increase physiological performance, and indirectly reduce the overall risk of all injuries, including concussions. They are also used in rehabilitation and alternative preventative treatment options. The gains in oxygen, performance and reduction in injury risk are not a direct result of the disclosed appliances directly absorbing impact energy or chemically altering one's physiology. Instead, these improvements are mainly achieved as a result of the athlete or individual being able to increase oxygen to the body, gain more efficiency of the skeletal, neurological and autonomic systems, and empower people to maintain peak power, endurance, and cognitive function longer. These and other objects, features, and advantages of the present invention will become apparent to those having ordinary skill in the art upon reading this disclosure.

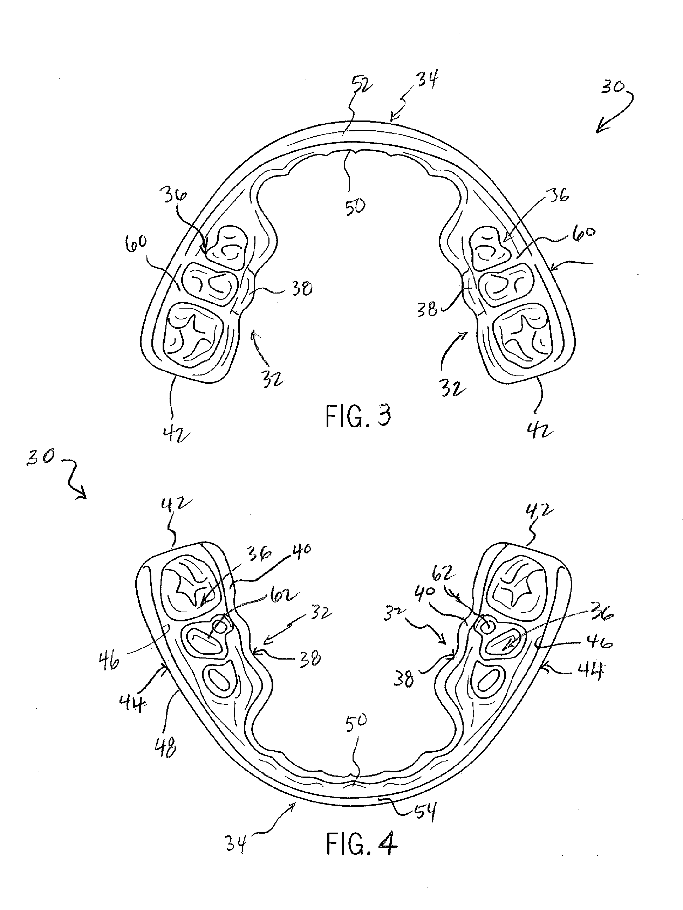

Turning now to the drawings, one example of the bite regulator or oral appliance constructed in accordance with the teachings of the present disclosure is illustrated in FIGS. 1-6. The oral appliance in this example is intended to fit over only portions of the bottom teeth of a wearer and is thus identified herein as a lower bite regulator 30. The lower bite regulator 30 has a pair of rear left and right molar sections 32 and a front band 34, or in this example a labial band interconnected to the rear molar sections at left and right ends of the labial band. The lower bite regulator 30 is unique in comparison to prior known oral appliances, mouth guards, and the like in a number of ways.

Each of the molar sections 32 has a top, relatively thick interocclusal region or portion 36 that is configured to rest between the molars of the wearer. Each molar section 32 also has a lingual wall 38 on an inner side or edge of the respective molar section. Each lingual wall 38 in this example depends downward from the respective molar section 32 and is contoured in two ways. First, the lingual wall 38 has a relatively thin wall thickness and is contoured (both inside/tooth facing side and outside/tongue facing side surfaces) to match the lateral, inner, or lingual surface contour of the adjacent molars. Second, the lingual wall 38 has a lower edge 40 that is contoured vertically so that the wall terminates at and follows the inner facing side or surface contour of the molars and does not contact any soft tissue or otherwise overlap any of the gums of the wearer. Each molar section 32 also has a short depending rear lip 42 that hangs over, in this example, a rear edge of the third (not the fourth or rearmost) molar between the third and fourth molars on each side of the wearer's mouth.

Each molar section 32 also has a buccal wall 44 on an outer side or edge of the respective molar section. In this example, the buccal walls that faces outward toward the wearer's cheek. In one example, an inner surface 46 of the buccal wall 44 can be contoured to conform to and closely follow the curvature and contour of the outer facing side of the wearer's molars. A buccal facing side or outer surface 48 of the buccal wall 44 can be relatively smooth. In another example, the outer surface or buccal facing side 48 of the buccal wall 44 could instead be contoured to generally match the contour of the inner surface 46. In such an example, the comfort for the wearer may be increased and the overall buccal wall thickness reduced to save material usage. In either example, the buccal walls 44 depend from the respective molar section 32 because the bite regulator 30 is worn on the lower teeth of a wearer. Each buccal wall 44 can have a height sufficient to cover a substantial portion of the wearer's soft tissue or gums on the buccal side of their lower teeth. In another example, in order to save material, each buccal wall can instead be contoured vertically so that the wall terminates at and follows the contour of the outer facing surfaces of the molars and does not contact any soft tissue or otherwise overlap any of the gums of the wearer.

The front band in this example is referred to as a labial band in that it is positioned on only the forward facing or labial side of the wearer's front lower teeth and interconnects the two molar sections 32. A rear side or surface 50 of the labial band 34 in this example is also contoured to custom fit or closely follow the contour of the forward facing labial surfaces of the wearer's front teeth. The height of the labial band 34 is such that a top edge 52 terminates at about the top edge of the wearer's lower front teeth but does not extend over the top edge. A lower edge 54 of the labial band terminates at about the wearer's gum line. Again, the lower edge 54 of the labial band 34 can be contoured so that no portion of the labial band extends downward to contact the soft tissue or otherwise overlap any portion of the soft tissue or gums of the wearer's mouth. In fact, the lower edge 54 of the labial band 34 can terminate above the gum line, if desired.

The lingual wall 38 of the molar sections 32 can include a comparatively thin wall thickness in comparison to prior known mouth guards. This can save on material usage, reduce obstruction in the wearer's airway, reduce distraction to the user wearing the device, and increase overall comfort for the wearer. Since there is less material covering the inner surface of the teeth, there is less obstruction between their tongue and the lower bite regulator during use, opening the airway and increasing comfort. The lingual wall 38 on the molar sections 32 is relatively thin because it is on the inside of the wearer's teeth and not on the outside. The lingual wall 38 of the molar sections 32 also can include an optional tongue shelf 56 in the form of a recess, depression, or scallop along the lingual wall, as shown in FIG. 7. The tongue shelf 56 can be provided to assist in properly locating the tongue for the wearer during use. The tongue shelf 56 will naturally allow the tongue to move forward, further opening the airway and providing a comfortable position resting on the lower bite regulator 30 while the device is being worn. Proper and relaxed tongue positioning will further increase oxygen intake and reduce required energy or stress on the wearer.

The rearward depth of the molar sections 32 on the lower bite regulator 30 is such that it does not cover all four of the molars on either side of the wearer's mouth. Instead, the molar sections 32 are truncated in comparison to prior known mouth guards, mouth pieces, oral appliances, and the like. Those prior known devices typically cover all of the wearer's molars and thus extend further rearward into the mouth and require more material to manufacture. See FIGS. 8A-8C, which show comparisons between the disclosed lower bite regulator 30 and an existing oral appliance device. FIG. 8A shows a perspective view of the lower bite regulator 30 on a set of lower teeth with the larger dimensions of existing devices depicted in the darker regions in the drawing. FIG. 8B shows a front view of a prior known oral appliance or lower mouth guard on a set of lower teeth of a wearer. FIG. 8C shows a front view of the lower bite regulator 30 on a set of lower teeth of a wearer. With the material on prior know devices extending further rearward into the wearer's mouth and further downward onto the soft tissue of the wearer's mouth, comfort is decreased, tongue interference is increased, and airway obstruction is increased. In contrast, the lower bite regulator 30 as disclosed herein uses significantly less material, eliminates this uncomfortable rear obstruction in the wearer's mouth, is much less intrusive so as to be less of a distraction to the wearer, and opens the airway for the user while wearing the device.

The top surfaces 60 of the interocclusal portions 36 of the molar sections 32 are custom molded to fit and engage the bottom facing side of the upper molars of a specific wearer or subject. The underside surfaces 62 of the interocclusal portions 36 of the molar sections 32 are custom molded to fit and engage the top side of the bottom molars of the specific wearer of subject. As described below, the positioning of these corresponding, mating depressions in the interocclusal portions 36 of the molar sections 32 is precisely fitted such that the wearer's upper and lower jaws, i.e., their bite alignment is registered and positioned for their correct or voluntary bite position, i.e., their natural and symmetrical neuromuscloskeletal alignment during use. When the wearer installs the lower bite regulator 30 and bites down on the device, their upper and lower jaws and teeth will register with one another at their correct natural or voluntary bite position and thus be in the most natural and comfortable alignment. This can significantly reduce stress in the wearer's jaw, resulting in reduced stress on the underlying parasympathetic and sympathetic systems and physiology of the wearer. During use, the wearer will naturally maintain a closed or clenched jaw position.

The disclosed lower bite regulator 30 is unique in that it still provides significant interocclusal impact protection in the same manner as a mouth guard while significantly reducing the size, material usage, and negative impact of wearing such an appliance in one's mouth during strenuous and stressful activity. See FIGS. 9A and 9B, which show a cross-section of the molar section and the labial band. The disclosed lower bite regulator 30 provides unobstructed breathing while the device is worn, allowing the wearer to consistently fuel the body, brain, and blood stream even under stressful and strenuous activity and stimulus. The disclosed lower bite regulator 30 helps to maintain a natural bite position and jaw registry for the user as well. The optional tongue shelf 56 of the lower bite regulator 30 minimizes the subconscious reaction to a foreign obstruction being placed in the mouth and helps to naturally locate or place the wearer's tongue in a relaxed, natural position within the mouth during use. The contouring on the disclosed lower bite regulator 30, and particularly the contouring on the lingual walls 38 of the molar sections 32, and the complete lack of a lingual wall on the labial band 34 in this example, significantly improves the wearer's ability to breathe and to communicate while wearing the device. See FIGS. 9A and 9B. The disclosed lower bite regulator 30 only employs thicker material regions where necessary, and particularly on the interocclusal portion 36 of the molar sections 32 between the wearer's upper and lower teeth, as depicted in FIG. 10.

The disclosed lower bite regulator 30 can be fabricated from any suitable material such as polymers or plastic. The disclosed products can also be made from more than one material, such as in a dual molding process. Likewise, the disclosed lower bite regulator 30 can be made and formed with different colored portions, clear portions, logos, and the like to enhance the aesthetic appearance and overall performance of the product. Material inserts can be fabricated during the molding process and placed, for example, in the interocclusal portions to provide further impact performance or absorption characteristics, if desired. The disclosed lower bite regulator 30 and other by regulators described herein are not intended to be limited to use of any particular material.

FIGS. 11-17 show another example of a lower bite regulator constructed in accordance with the teachings of the present disclosure. In this example, the lower bite regulator is constructed essentially the same as the prior described lower bite regulator except in the region of the labial band. In this example, like reference numerals refer to like parts of the lower bite regulator 30. In this example, the lower bite regulator is referred to as a hybrid lower bite regulator 70 because it provides some mouth guard functionality. Specifically, the hybrid lower bite regulator has a front band 72 with a labial wall 74 and a lingual wall 76 that replaces the labial band 34 of the above-described lower bite regulator 30. The front band 72 in this example is configured to fully cover both the labial side and lingual side of the wearer's front teeth and thus is generally U-shaped in cross-section and extends over the top edge of the front teeth. See FIG. 17.

The lingual wall 76 can be a continuation of the molar section lingual walls 38 and labial walls and the labial wall 74 can be substantially similar to the labial band 34 of the prior example. The interior surface 78 of the labial wall 74 and the interior surface 80 of the lingual wall 76 of the front band 134 in this example are each custom fitted and contoured to match the labial and lingual surface contours of the wearer's teeth. The lingual wall 76 of the front band 70 can again have a substantially thin wall thickness and can be contoured to minimize obstruction with the front of the wearer's tongue during use.

The lingual wall 76 can optionally be provided with a tongue shelf 82 that aligns with the earlier described tongue shelves 56 on the molar sections 32 to further assist in locating the wearer's tongue in a natural position while wearing the device. Alternatively, the lingual wall 76 of the front band 72 in this example can have a bottom edge that terminates above the soft tissue or gum line of the wearer and/or is contoured so as to not contact any soft tissue or otherwise overlap the gum line of the wearer. Such a limited lingual wall would likely not accommodate much of a tongue shelf. The labial or outer facing surface of the labial wall 74 in this example can be smooth or again can be contoured if desired. Providing a smooth outer surface would allow for increased wall thickness on the labial side of the labial band, which can offer better tooth protection and, thus, mouth guard functionality. This may be desirable in some circumstances because the device is a hybrid device configured to cover both sides of the front teeth and may be specifically intended to offer some mouth guard functionality.

FIGS. 18-23 show another example of a bite regulator constructed in accordance with teaching of the present disclosure. In this example, the oral appliance is configured as an upper bite regulator 90 and is to be received on and conform to the upper teeth of a wearer and not the lower teeth. In general, the upper bite regulator 90 is configured similar to the earlier described lower bite regulator 30 and hybrid lower bite regulator 70, but is more similar to the lower hybrid bite regulator in that it functions in part at a mouth guard. The upper bite regulator 90 has left and right molar sections 92 that have essentially the same construction as the earlier described molar sections 32. However, no tongue shelf is provided, as the tongue would not be in contact with the upper device. The molar sections 92 are connected to one another by a front band 94 with left and right ends connected to the molar sections 92. Each molar section 92 has a thick interocclusal region 96 that would be positioned between the upper and lower molars of a wearer. Each molar section 92 has a top surface 97 and a bottom surface 99 that are again molded to match the contour of the wearer's upper and lower molars.

The molar sections each have a lingual wall 98 extending up from an inner edge. The lingual walls 98 can have a bottom edge 100 that is contoured to not touch, overlap, or otherwise contact the soft tissue of a wearer. The lingual walls are also contoured (both inside/tooth facing side and outside/tongue facing side surfaces) to match the contour of the adjacent molars. The molar sections 92 also can each have a rear lip 102 that protrudes up from a rearward most free end of the molar sections. Each molar section 92 also has a buccal wall 104 protruding up from an outside edge of the molar section. The inside or teeth facing surfaces 106, 108, respectively, of the lingual and buccal walls 98, 104 can be contoured to match that of a specific wearer or subject, as with the previous examples. The molar sections 92 are again designed to terminate at the third molar, not the fourth or rearmost molar and the rear lip is intended to lay against a rear surface of the third molar and reside between the third and fourth molars.

The primary difference between the upper bite regulator 90 and the lower models described above is that the upper bite regulator is configured to fit over the upper teeth of the wearer. Also, the front band 94 can be configured to provide added dental coverage and protection, imparting the addition of mouth guard functionality to the device. The front band 94 in this example can have a lingual wall 110 and a labial wall 112 joined to one another at a bottom edge 114, similar to the hybrid lower bite regulator 70, but for the upper front teeth. The lingual wall 110 can have a substantially thin wall thickness and can be contoured to match the lingual face contour of the wearer's teeth. The bottom edge 114 of the front band 94 in this example is intended to wrap around the lower edge of the wearer's upper front teeth. See FIG. 24. An upper edge 116 of the lingual wall 110 on the front band 94 can be contoured so as not to contact the soft tissue or otherwise overlap any portion of the wearer' gum line. An outer or labial surface 118 of the labial wall 112 on the front band 94 in this example can be smooth so as to allow for a more robust wall thickness covering the wearer's front teeth to enhance the mouth guard functionality of the device. The labial wall 112 can also extend upward and covers a good or substantial portion of the soft tissue or gums above over the wearer's front teeth.

FIG. 25 shows a comparison of the upper bite regulator 90 to a more conventional oral appliance of mouth guard construction. The dark areas in the drawing represent material found on the conventional device that is eliminated on the upper bite guard 90.

On the hybrid lower bite regulator and the upper bite regulator, the interocclusal portions 36, 96 of the molar sections 32, 92 again have a much thicker wall thickness to provide impact protection. The top surfaces 60, 97 and underside or bottom surfaces 62, 99 of the respective occlusal regions 36, 96 of the molar sections 32, 92 are also custom fitted, molded, and bite registered to provide the bite registration noted above and described further below.

The disclosed bite regulator models offer high performance as a custom mouth piece. The disclosed bite regulator models are light, flexible, custom fitted polymer plastic mouth pieces that perfectly regulate a person's bite to provide enhanced breathing, clear communication, and proper skeletal alignment during high impact and high stress activities and in such environments. The increase in oxygen naturally increases both performance and protection by allowing athletes to train harder, recover faster, react quicker, avoid fatigue longer, and make better decisions when it matters most.

The PX3 bite regulators are proven to increase oxygen volume, dopamine levels, heart rate variability and reduce concussions. Numerous test and studies to date have proven that the superior performance of the bite regulators.

The fundamental principles of the disclosed bite regulators are: greater oxygen equals greater performance equals greater protection. It doesn't get much simpler than that and yet no such device or product has previously been developed, until now. The PX3 bite regulators disclosed herein are proven performance enhancing mouth pieces or oral appliances that achieve real, consistent, and measureable results with all athletes and body types during high impact activities and in high stress environments. It is well researched and documented that oxygen levels and brain function go hand in hand, affecting motor skills, vision, hearing, reaction time, strength, flexibility, balance, the ability to learn, heal, and protect, plus thousands of other neurological responses. It is very well researched and documented that jaw position affects the flow of oxygen to the body. CPR training and certification instructs people to bring the patient's jaw forward to open up the airway. You would never push the jaw back in the throat, or move it all the way to the left or right without significantly occluding the airway.

FIG. 26 shows the skull and neck of a person with arrows depicting how jaw misalignment can negatively affect performance. Neck and back tension begin with an unbalanced bite. With good posture, one's head exerts an 8-16 pound pull on the neck muscles in the direction of the arrows P. The arrow B shows the force direction exerted when the person's bite is correctly aligned and balanced. The arrow U shows the force direction exerted when the person's bite is incorrect and out of balance. Under such unbalanced conditions, the head can exert a 30 pound pull force on the neck muscles causing strain and tension.

In sports, the jaw is always in motion. In high impact sports, the jaw is constantly being knocked around and misaligned through impact. Regardless of impact, even just with a high stress environment, the natural tendency for people is to clench down and stop breathing all together. Knowing the role that the jaw plays and how important oxygen is to overall human function, the disclosed PX3 bite regulators have been developed to capture a person's unique and optimal breathing position and regulate the jaw in that position so that it prevents it from sliding or causing postural skeletal misalignment during high impact and high stress environments. In sports, these are typically the most crucial moments when success and failure truly matter most. In other areas of life, such as in hostile military combat, these moments are when success and survivability are defined.

The disclosed bite regulators have been developed with power, protection and performance in mind. The bite regulators have been extensively tested and developed in the most extreme high performance environments in the world. The bite regulators significantly advance this field in three key areas:

1. Reducing the risk of concussions and career ending injuries

2. Eliminating the need for Illegal Performance Enhancing Drugs

3. Optimizing Health and Wellness

The PX3 research council is one of the most comprehensive in sports and is made up of leading Neurologists, Physiologists, Kinesiologists, Medical Doctors, Chiropractors, Dentists, Athletic and Military Special Ops trainers, and the like. The bite regulators that have been developed have surpassed expectations in performance in these key areas.

The lower bite regulator 30 is custom fitted to lower teeth and is best in class for quality, comfort, and durability. The lower bite regulator 30 offers the highest volume of oxygen increase for these types of products. The lower bite regulator 30 also offers the clearest ability to communicate while wearing the device. The tongue is left free to move so that a wearer can speak nearly normally, even with their jaw clamped down on the device. The lower bite regulator 30 is designed for all sports, training, and physical activities where full dental coverage is not mandated.

The lower hybrid bite regulator 70 provides full dental coverage of the lower teeth for contact sports that require mouth guards while still providing all of the characteristics and benefits of the lower bite regulator 30. The lower hybrid bite regulator 70 is designed specifically for youth contact sports, such as football, ice hockey, lacrosse and field hockey, where mouth wear that provides full dental coverage of either the top or bottom teeth is mandated, but where the athletes have additional facial protection provided by the sport specific head gear. The upper hybrid bite regulator 90 provides full dental coverage of the upper teeth for contact sports that require or mandate full dental protection while still also providing all of the performance characteristics and benefits of the lower bite regulator 30.

Each of the bite regulator models is contoured on the lingual side, cut out around each individual tooth to have minimal or no contact with the lingual soft tissue. The lower and lower hybrid models can also have the added feature of additional contouring to include the tongue shelf for additional airway opening. The smooth outside or labial surface contouring can be important for long-term use and overall comfort. The thin wall thickness and contouring on the lingual walls and surfaces are also important for long term us, very comfortable fit, and increased ability to speak and breathe while wearing the device.

Tongue position can significantly affect one's airway. When the tongue comes into contact with anything in the mouth, it has been shown to naturally retract back into the throat, which occludes the airway. The tongue shelf on the lower models allows the tongue to push forward and rest comfortably, opening the air way and resting tongue muscles. The labial band or front band on the lower and lower hybrid bite regulator models comes up off the front gums and rests only on the teeth for added comfort and extended wear.

The bite regulators have been tested and proven to enhance Neuromuscular, Physiological, Neuromusculoskeletal, and Neurological performance. Every single millimeter on these types of devices has been found by the patentee to make a profound difference on performance and comfort. The disclosed bite regulators are totally unique and greatly benefit from highly skilled and trained lab technicians using state-of-the-art tools and technology fabricating and final fitting the devices.

The disclosed bite regulators greatly enhance performance by perfectly contouring the lingual side (upper and lower models) so as to stop at and follow the natural/unique tooth/gum line (vertical up and down) and by factoring in the natural/unique contours of the patient's teeth (horizontal in and out/side to side). On the lower bite regulator, the labial band is cut away almost completely, just barely covering the front 6 teeth. All models only go as far back as the third molar. On the lower guards, the tongue shelf maximizes air flow through optimized tongue alignment.

According to the Centers for Disease Control, as many as 3.8 million athletes suffer a concussion each year in the US. Even more alarming, traumatic brain injury (TBI) is the leading cause of death and disability in persons under 45 years old, occurring more frequently than breast cancer, AIDS, multiple sclerosis, and spinal cord injury combined. Other equally revealing statistics are that:

[0069] - Brain injury is suffered by someone in America, usually a young person, every 15 seconds;

[0070] - Each year, approximately 100,000 people die from TBI and 500,000 more are permanently disabled;

[0071] - 80,000 people experience the onset of long-term disability following a severe brain injury annually; and

[0072] - The cost of treating, rehabilitating and caring for the victims of traumatic brain injury costs the U.S.

over $50 billion each year.

Concussion testing and research are focused on the equipment and force or impact levels. It is also well documented that mouth pieces have little effect in reducing concussions or the severity of concussions from an impact.

It is also well documented that most injuries happen in the second half of the game, and the second half of the season. This has driven the patentee to reevaluate the concussion issue. The patentee has considered and determined that if knows that the integrity of the protective equipment is constant, and that impact levels over the course of competition are constant, then the physical and mental state of the athlete must be the major variable. Countless studies and programs continue to focus on the equipment and the force of impact, looking for a possible solution. The patentee has noticed that these tests are one dimensional, measuring external force exerted on the athlete or on the equipment and simply not relevant to the complexity or in any way measuring the internal neurological or physiological components of what is truly involved in these types of injuries.

The patentee has noted that mouth guards restrict breathing and misalign the jaw. This wears athletes down faster, making them less efficient and increasing the risks of concussions rather than having any preventative impact on reducing these risks. In fact, it has been noted that the use of mouth guards increased the frequency of concussions. The patentee's research clearly shows how mouth guards restrict breathing and negatively impact athletes on just about every physical, physiological, and neurological level. Using very basic kinesiology testing, the patentee has also clearly sees how athletes are weaker from a strength, balance, and flexibility standpoint. Mouth guards, even custom fitted mouth guards, appear to the patentee to be a bigger part of the problem than anyone has heretofore realized. The disclosed bite regulators are not mouth guards. In fact, they are the exact opposite from a neurological and physiological performance standpoint in the proven fact that the bite regulators enhance these factors rather than limit or restrict them.

The disclosed bite regulators are also not therapeutic orthotic devices. Therapeutic orthotic devices are designed to treat disorders. Such devices are not designed for unobstructed breathing or communication, are not used in high impact athletic or military environments, and are not proven to reduce concussions. Orthotics are positioned where all masticatory muscles, including all antagonistic muscle groups, such as elevators and depressors, are in the state of minimal electrical activity necessary to maintain postural rest. Orthotics also require K-7 and TENS technology, a 90 to 120 minute fitting exclusively by neuromuscular trained dentists, which account for only less than 3% of the entire dental population.

In contrast, one example of a fitting process for the disclosed bite regulators can take less than 15 minutes. In one example, a custom manufacturing and self-impression series kit can be used to fit the regulators to a specific user. One example of such a kit 128 is shown in FIGS. 27 and 28. In one example, the kit 128 can include bite trays or fitting trays including an upper tray 130 and a lower tray 132. The kit 128 can also include impression putty 134 that is used to take the critical impressions of the person's teeth. The kit 128 can also include instructions 136 and one or more shipping bags 138 and/or containers 140 for purposes described below. In accordance with the teachings of the present invention, the kit 128 can also include a power bite simulator or bite fork 142 that is used to specifically determine and record the bite registry of the person taking their own impressions.

In another example, a person intending to get fitted for a bite regulator as disclosed and described herein can visit a professional facility, either a dentist or a dedicated fitting facility, where a professional will conduct the fitting, take impressions, determine the bite registry, and the like. That facility can then either manufacture or fabricate a bite regulator on-site, if capable, the bite regulator specifically fitted for that person or can send the various impressions to a dedicated facility that manufactures the bite regulators. Dentists can become certified and trained to perform the various fitting techniques so that the correct jaw alignment can be determined for each individual. Dentists can also offer advanced fitting techniques for patients that have orthodontics or suffer from severe jaw disorders or disfiguration.

With the self-impression series kit 128, the user can readily perform the various fitting steps and alignment steps on their own. FIG. 28 illustrates one example of a bite fork 142 that is used in accordance with the teachings of the invention to determine each person's proper jaw alignment and natural jaw position. The bite fork 142 has a handle 144 at one end and an arcuate or curved section 146 connected to the handle. The curved section 146 is configured to be received in the person's mouth during use. The bite fork 142 also has a plurality of notches 148a-f formed on both the top side 150 of the fork between the handle 144 and the curved section 146 and on the bottom side 152 as well. The top side 150 includes two notches 148a, 148b in this example for providing two different landing locations for a user to place their upper teeth. The bottom side includes four notches or landing zones 148c, 148d, 148e, and 148f in which the user can place their lower teeth. Each of the upper notches is spaced at a different vertical distance to the lower notches. The user can thus also determine their upper to lower jaw spacing position that is most comfortable. In one example, the spacing between the upper and lower notches can be 3.5 mm for one of the upper notches and 4.0 mm for the other of the upper notches. A range of different thickness bite forks can also be provided, such as accommodating between a 2.0mm vertical opening to a 5.0mm vertical opening. Other examples are certainly possible.

For example, FIGS. 29-31 show three such examples. FIG. 29 shows a Class I bite fork 160 that has a single upper notch 162 and a single lower notch 164 to accommodate people having a relatively normal or vertically aligned bite. FIG. 30 shows a Class II.III bite fork 170 that has one upper notch 172 and one lower notch 174 that are vertically misaligned. In FIG. 30, the bite fork is oriented to accommodate a Class II overbite. In FIG. 31, the same bite fork is flipped over to accommodate a class III under-bite. The bite forks 160 and 170 can be provided in a range of thickness so that the subject can find their optimal jaw/bite opening and can be provided in a range of vertical misalignment between the notches, if desired. As will become evident to those having ordinary skill in the art, the features of the bite forks can be modified to accommodate a wide range of individuals. The goal is to create a bite regulator that accommodates the subject's natural jaw position both fore-aft and vertically.

During use, a user will place their upper teeth in one of the upper notches of one of the bite forks and place their lower teeth in a most comfortable one of the lower notches of the same bite fork. Trial and error might be necessary to find the most comfortable, i.e., correct jaw position. The subject can try this for either of the two upper notches 148a, 148b of the fork 142 for example to determine the most comfortable vertical spacing and the most comfortable fore-aft position of their lower teeth relative to their upper teeth.

Methods of fitting are described below and one example of same is shown in the flow charts of FIGS. 32-34. Generally, the entire kit 128 is provided to the user in one of the bags 138 or containers 140. The user removes the trays 130, 132, impression putty 134, bite fork(s) 142, and additional bags 138 from the primary bag. The user then performs the process of taking their impressions, accounting for their correct jaw position, and having the regulators made accordingly, as described below. Once the fitting process is completed, the user in one example places each separate impression device (trays 130, 132 and formed putty 134) in a separate return shipping container 140 or bag 138, and then ships their impressions back to the manufacturer so that the bite regulator can be fabricated.

In one example, each user will utilize both the upper and lower impression trays 130, 132, regardless of whether they are looking for a lower bite regulator, hybrid lower bite regulator, or upper bite regulator. As a first step, the user should heat each of the trays separately (e.g. boil in water for 60 seconds) and bite into the trays (e.g. for 30 seconds) in order to generally conform or shape the trays to their teeth and mouth shape. The trays can be placed in cold water for one second if needed and depending on the tray material. If the trays are too large, the trays can be trimmed as needed to fit the mouth of the user. Each of the trays can be fabricated from material that will allow leaving some impression in the tray surfaces and will alter the shape of the tray to generally conform to the user's mouth.

The next step is to mix the impression putty 134 as needed and depending on the composition of the putty. The putty is then rolled and formed and placed in the preformed impression tray. The putty can be formed so it is flush to the outer edges of the trays. Each tray is done separately. The user will then place one of the trays (upper or lower) in the mouth that has been loaded with the impression putty. Once inserted, the user should bite down and hold their position for as long instructed (e.g. for 3 minutes) for the impression putty to properly set. The next steps are to do the same for the other tray, whether upper or lower, so that the user has obtained an impression of both their upper teeth and their lower teeth.

The next step is to determine the bite registration for the particular user. The bite fork(s) are utilized for this purpose. The user must first determine their most comfortable jaw position, and spacing if the aforementioned bite fork 142 is utilized, prior to creating the impression. The user can practice by placing their teeth in various ones of the notches and performing simple physiological tests per the instructions 136, check their breathing, and the like in order to determine the best position for them. During the next step, the user again is required to mix and form the impression putty 134 and roll and form the mixed putty. In this example, the user is to separate the putty into two portions and place one portion on each side of the arcuate or curved section 146 of the bite fork. Once the putty is in place, the user is required to stand in front of a mirror, position their upper teeth in the correct notch position, if applicable (unless already using a specific single notch fork geometry) and then slowly bite into the device, making sure their lower teeth land in the predetermined notch positions. Once the user's teeth and jaw are in place, the user is to bite down on the device and hold a suitable amount of time for the impression putty to set. The user can also push on their lips and cheeks with their hands and on the inside with their tongue to better shape and form the putty to their mouth and teeth shape.

Once all of these impression steps are completed, the user can package the three impressions in the return container 138 or 140 and ship the impressions to the manufacturer. The manufacturer will then, based on these three impressions, make molds that exactly match the user's teeth impressions and then determine the precise jaw and bite registry for the particular user. The manufacturer will then custom fabricate and fit a bite regulator to fit their upper teeth or their lower teeth, depending on the model selected, and their natural bite registry. Once the bite regulator is fabricated, it can be return shipped to the user as a completed product. The manufacturer can retain impressions so that the user can order additional products, if and when necessary.

The process of making and fitting the disclosed bite regulators can vary from the example described herein. For example, the power bite simulator or bite fork can include or be used as one of the upper or lower trays, eliminating one of the trays. In such an example, the user will need only take two impressions, one using the lone tray (upper or lower) and one using the combination bite fork/tray (the other of the upper or lower not yet taken). The types of trays can also be varied as well and can be any suitable commercially available tray or a specially developed tray shape and/or material. Specially designed hard (non-formable) trays could also be used, as can standard hard trays, standard mesh trays, and other custom trays. Digital scanners can also be successfully used in this process. A digital scanner can take all the necessary measurements for the nine separate factors noted above, while the patient's jaw is properly aligned.

In other alternatives, the process to have the bite regulators fabricated can also vary. For example, if a digital scanner were used, it can be used to scan the upper and lower arches of the teeth. The subject can then use the bite fork to obtain the correct jaw position. The scanner can then be used to scan the labial side of the upper and lower arches of the teeth and gums. The scanner can electronically save the data in a file, which can be e-mailed to the designated PX3 lab or approved lab for fabrication.

If a professional dental care technician is taking the impressions, the technician can take a standard upper arch impression using impression material, i.e., putty, and using either a metal or thermoplastic impression tray. The technician can then take a standard lower arch impression using impression material and either a metal or thermoplastic impression tray. The bite fork can then be loaded with impression material to capture and obtain the upper and lower teeth in order to establish the proper bite position. The impressions and bite registration impression can then be shipped by the dental technician to the PX3 designated lab or approved lab.

If the subject is using the self-impression process, the subject can mix impression material together, load one side of the bite fork, bite down to the fork, and then sculpt/mold the impression material around their teeth and gums on the lingual side with their tongue and on the labial side with their fingers and cheek pressure. The subject can then mix impression material together, load the other side of the same bite fork or a separate bite fork, and then sculpt/mold the impression material around their teeth and gums on the opposing lingual side with their tongue and the labial side with their fingers and cheek pressure. The subject can then ship the double impression/bite registration to the designated PX3 lab or approved lab. The two impressions should overlap in the middle so that a full impression can be matched and made from the two separate impressions.

No one has heretofore developed or offered a self-impression system and fully customized bite regulator mouth piece that registers the top teeth, bottom teeth, vertical spacing on the left, right and middle occlusal with kits or steps. The self-impression or professional fitting process can also utilize the aforementioned digital process or a two-step impression process instead of the three step process described in detail herein.

Pro (Dentist – impression + bite fork)

SIS (Patient –impression + bite fork)

Dentist Fitting Process: 2 steps
Upper impression or Lower impression
PX3 bite fork to register the bite
SIS Fitting process: 2 steps
Upper impression or Lower impression
PX3 bite fork to register the bite
In one example, the bite regulator models can have the following dimensional characteristics. It should be noted that the invention is not to be limited to the following examples.

**Lower Bite Regulator**

Buccal extension - 3 mm apical from CEJ
2.5 mm thick
Flat, no contouring
Lingual extension - 1 mm apical of CEJ
lmm thick
Embrasure contouring begins 3 mm apically from occlusal table
Feather edge lingual termination
Guard terminates at the mesial half of second molar
exception: if there is no second molar then termination ends on first molar
if there is no first molar, a first molar can be created in the guard
Round off distal lingual of last tooth covered by guard
Open/ Flatten off buccal aspect of cuspid index
Occlusal index
.5 mm on lingual cusps
1 mm on buccal cusps
.5 mm on lingual and buccal cusps of last tooth indexed
Labial connector
Polished intaglio, so there is little to no contact to hard and soft tissue
4 mm width from CEJ to incisal edge
Contoured when needed at labial frenum
Cuspid coverage extends no more than half of the tooth
Lingual cuspid concavity
No indexing past cuspids
Tongue Shelf
small concavity created on lingual
concavity extends throughout whole lingual surface posterior to anterior
concavity begins at occlusal table and extends only to beginning of embrasure line
concavity size is determined on the amount of material built on occlusal table. Some guards may have longer concavities due to space from occlusal table to embrasure line.
Lingual contouring
**Hybrid Upper Bite Regulator**
Buccal extension - 2 mm apical from CEJ
2.5 mm thick
Flat, no contouring
Palatal extension - 1 mm apical of CEJ
1 mm thick
2-3 mm from incisal edge to cingulum of anterior teeth (cuspid to cuspid)
Embrasure contouring begins 3 mm apically from occlusal table
Feather edge palatal termination
Guard terminates at the mesial half of the second molar
exception: if there is no second molar then termination ends on first molar
if there is no first molar a first molar can be created in the guard
Round off distal palatal of last tooth covered by guard
Open/Flatten offbuccal aspect of cuspid index
Occlusal index
.5 mm on lingual cusps
1 mm on buccal cusps
.5 mm on lingual and buccal cusps of last tooth indexed
Cuspid palatal coverage extends no more than half the tooth
No indexing past cuspids
Lingual contouring
The disclosed fitting process can take you less than 15 minutes. The entire custom manufacturing and shipping processes can take up to 3 weeks depending on where the person resides. The disclosed custom fitting process of the bite regulators is also completely unique.
Bite regulators are disclosed herein and numerous features and design aspects are disclosure for such bite regulators. Various combinations of the features can be provided on a given bite regulator. Any one of the disclosed features and design aspects may be provided either separate from or in combination with any one or more of the other features and design aspects, where possible, even though that particular combination or arrangement is not specifically disclosed or described herein.
Although certain oral appliances, components, and methods have been described herein in accordance with the teachings of the present disclosure, the scope of coverage of this patent is not limited thereto. On the contrary, this patent covers all embodiments of the teachings of the disclosure that fairly fall within the scope of permissible equivalents.

## Claims

1. A bite regulator comprising:

   a front band with left and right ends; and
   left and right molar sections extending rearward respectively from the left and right ends of the front band,
   wherein the left and right molar sections each have an interocclusal region with a contoured top surface and a contoured bottom surface, and wherein the contoured top and bottom surfaces are each configured to match a specific contour of the wearer's top and bottom teeth, respectively, and are aligned relative to one another to correctly position the wearer's jaw.

2. A bite regulator according to claim 1, wherein the front band is a labial band configured to contact only an outer facing side of a wearer's front teeth.

3. A bite regulator according to claim 2, wherein the labial band has a tooth facing inner surface that is contoured to match a front surface contour of the wearer's front teeth.

4. A bite regulator according to claim 2, wherein the bite regulator is a lower bite regulator and a top edge of the labial band terminates at or below a height of a user's bottom front teeth.

5. A bite regulator according to claim 2, wherein the bite regulator is a lower bite regulator and a bottom edge of the labial band is contoured to follow a soft tissue line between the gums and the wearer's front teeth but not overlie the soft tissue.

6. A bite regulator according to claim 1, wherein the left and right molar sections each have an interocclusal region with a contoured top surface and a contoured bottom surface, and wherein the contoured top and bottom surfaces are each configured to match a specific contour of the wearer's top and bottom teeth, respectively, and are aligned relative to one another to correctly position the wearer's jaw.

7. A bite regulator according to claim 1, wherein the left and right molar sections each have a lingual wall on an inner side of the respective molar section, and wherein a tongue shelf is formed on each of the lingual walls.

8. A bite regulator according to claim 7, wherein the bite regulator is a lower bite regulator and each lingual wall depends from the respective molar section and has a bottom edge that is contoured vertically to follow a contour of the soft tissue line of the wearer and does not contact or overlap any soft tissue below the molars of the wearer.

9. A bite regulator according to claim 1, wherein the left and right molar sections each have a buccal wall on an outer side of the respective molar section, the bite regulator is a lower bite regulator and each buccal wall depends from the respective molar section and has a bottom edge positioned below the soft tissue line of the wearer so that the buccal wall contacts or overlaps the soft tissue below the lower molars of the wearer.

10. A bite regulator according to claim 1, wherein the bite regulator is a hybrid lower bite regulator with the front band having a front wall portion that at least partially covers a front side of the wearer's lower front teeth and a rear wall portion that at least partially covers a back side of the wearer's lower front teeth.

11. A bite regulator according to claim 1, wherein the bite regulator is an upper bite regulator with a V-shaped front band having a labial wall and a lingual wall connected to one another along a bottom edge, the lingual wall having a top edge that is contoured vertically to follow a contour of the soft tissue line of the wearer and does not contact or overlap any soft tissue above the front teeth of the wearer.

12. A bite regulator comprising:

    a front band with left and right ends; and
    left and right molar sections extending rearward respectively from the left and right ends of the front band,
    wherein the left and right molar sections terminate short of a fourth rearward molar of a wearer.

13. A bite regulator according to claim 1, further comprising a rear lip protruding from a free end of each of the left and right molar sections, each rear lip configured to lie between a third and fourth molar of a user and against a rear edge of the third molar.

14. A bite regulator according to claim 13, wherein the bite regulator is a lower bite regulator and each rear lip depends downward from the respective free end of the corresponding molar section.

15. A bite regulator according to claim 13, wherein the bite regulator is an upper bite regulator and each rear lip protrudes upward from the respective free end of the corresponding molar section.

FIG. 1

FIG. 2

FIG. 3

FIG. 4

FIG. 5

FIG. 6

FIG. 10

FIG. 7

FIG.8B

PRIOR ART

FIG. 8C

FIG. 8A

FIG. 9A

FIG. 9B

FIG. 11

FIG. 12

FIG. 13

FIG. 14

FIG. 15

FIG. 16

FIG.17

FIG. 18

FIG. 19

FIG. 20

FIG. 21

FIG. 22

FIG. 23

FIG. 24

FIG. 25

FIG. 26

128

134

140

132

130

134

138

142

138

138

136

**FIG. 27**

142

146

150

148a

148b

144

148c

148d

148e

148f 154

**FIG. 28**

FIG. 29

FIG. 30

FIG. 31

```
┌─────────────────────────┐   ┌──────────────────────────┐
│   FIT  TOP  TRAY BLANK   │   │  FIT  BOTTOM  TRAY BLANK  │
│  TO  YOUR  TEETH  PROFILE│   │  TO  YOUR  TEETH  PROFILE │
└─────────────────────────┘   └──────────────────────────┘
              │                            │
              ▼                            ▼
   ┌──────────────────────────────────────────────┐
   │  PUT  TRAY  INTO  BOILING  WATER  FOR  60 SECONDS │
   └──────────────────────────────────────────────┘
                         │
                         ▼
   ┌──────────────────────────────────────────────────────┐
   │ IMMEDIATELY  PUT  TRAY  INTO  COLD  WATER  FOR  1 SECOND │
   └──────────────────────────────────────────────────────┘
                         │
                         ▼
        ┌──────────────────────────────────┐
        │ IMMEDIATELY  PUT  TRAY  INTO  MOUTH │
        │  AND  BITE  HARD  FOR  30 SECONDS   │
        └──────────────────────────────────┘
                         │
                         ▼
   ┌──────────────────────────────────────────────┐
   │ WHEN  FITTED  PROPERLY  THE  TRAYS  ARE  FINISHED │
   └──────────────────────────────────────────────┘
```

FIG. 32

```
┌──────────────────────────┐   ┌────────────────────────────┐
│ MAKE  TOP  TEETH  IMPRESSION│   │ MAKE  BOTTOM  TEETH  IMPRESSION│
│ USING  YOUR  FITTED  TOP TRAY│   │ USING  YOUR  FITTED  BOTTOM TRAY│
└──────────────────────────┘   └────────────────────────────┘
              │                            │
              ▼                            ▼
        ┌──────────────────────────────────────┐
        │ MIX  INGREDIENTS  TOGETHER  TO  MAKE  PUTTY │
        └──────────────────────────────────────┘
                         │
                         ▼
            ┌──────────────────────────┐
            │ MAKE  A  BALL  OF  THE  PUTTY │
            └──────────────────────────┘
                         │
                         ▼
            ┌──────────────────────────┐
            │ ROLL  PUTTY  INTO  A  CYLINDER │
            └──────────────────────────┘
                         │
                         ▼
   ┌──────────────────────────────────────────────────┐
   │ LOAD  PUTTY  INTO  TRAY  TILL  FLUSH  TO  OUTSIDE  EDGES │
   └──────────────────────────────────────────────────┘
                         │
                         ▼
      ┌────────────────────────────────────────┐
      │ INSERT  TRAY  WITH  PUTTY  INTO  MOUTH  AND │
      │  BITE  DOWN  AND  HOLD  FOR  3 MINUETS    │
      └────────────────────────────────────────┘
```

FIG. 33

FORM  BITE  REGISTRATION

↓

USING  THE  BITE  FORK  FIND  YOUR  COMFORTABLE  TEETH  POSITION

↓

MIX  INGREDIENTS  TOGETHER  TO  MAKE  PUTTY

↓

MAKE  A  BALL  OF  THE  PUTTY

↓

ROLL  PUTTY  INTO  A  CYLINDER

↓

CUT  ROLLED  PUTTY  INTO  TWO  EQUAL  HALVES

↓

LOAD  ON  TO  FORK  ENDS

↓

PLACE  FORK  WITH  PUTTY  INTO  MOUTH  THEN  SLOWLY
BITE  DOWN  WITH  TONG  APPLYING  INSIDE  PRESSURE
AND  YOUR  HANDS  APPLYING  OUTSIDE  PRESSURE
THROUGH  YOUR  CHEEKS  FOR  2  MINUTES

FIG. 34

MAKE  BITE  REGU-
LATOR

Europäisches
Patentamt

European
Patent Office

Office européen
des brevets

**EUROPEAN SEARCH REPORT**

Application Number

EP 14 16 0389

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2009/155223 A1 (BITE TECH INC [US]; ROETTGER MARK A [US]; MOLHOEK ROBERT C [US]; KITTE) 23 December 2009 (2009-12-23) * the whole document * ----- | 1-15 | INV. A61F5/56 |
| X | US 2006/011204 A1 (MAHER GERALD J [US]) 19 January 2006 (2006-01-19) * paragraph [0030] - paragraph [0035]; figures * ----- | 1,2,4-7, 12 | |
| X | US 2006/078840 A1 (ROBSON FARRAND C [US]) 13 April 2006 (2006-04-13) * the whole document * ----- | 1-10 | |
| X | WO 2011/159299 A1 (SPORT GUARD INC [US]; HACKMAN JOHN A [US]; QUINN BOBBIE [US]; ARMIDEO) 22 December 2011 (2011-12-22) * the whole document * ----- | 1-3,6,7, 11,12 | |

TECHNICAL FIELDS
SEARCHED (IPC)

A61F
A63B

The present search report has been drawn up for all claims

| Place of search | Date of completion of the search | Examiner |
|---|---|---|
| The Hague | 6 June 2014 | Sánchez y Sánchez, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 14 16 0389

This annex lists the patent family members relating to the patent documents cited in the above-mentioned European search report.
The members are as contained in the European Patent Office EDP file on
The European Patent Office is in no way liable for these particulars which are merely given for the purpose of information.

06-06-2014

| Patent document cited in search report | | Publication date | Patent family member(s) | | Publication date |
|---|---|---|---|---|---|
| WO 2009155223 | A1 | 23-12-2009 | AU | 2009260363 A1 | 23-12-2009 |
| | | | CA | 2728920 A1 | 23-12-2009 |
| | | | CN | 102123678 A | 13-07-2011 |
| | | | EP | 2331005 A1 | 15-06-2011 |
| | | | EP | 2659938 A2 | 06-11-2013 |
| | | | JP | 5221681 B2 | 26-06-2013 |
| | | | JP | 2011514185 A | 06-05-2011 |
| | | | WO | 2009155223 A1 | 23-12-2009 |
| US 2006011204 | A1 | 19-01-2006 | CA | 2573572 A1 | 23-02-2006 |
| | | | GB | 2431357 A | 25-04-2007 |
| | | | US | 2006011204 A1 | 19-01-2006 |
| | | | WO | 2006019934 A2 | 23-02-2006 |
| US 2006078840 | A1 | 13-04-2006 | NONE | | |
| WO 2011159299 | A1 | 22-12-2011 | US | 2012199141 A1 | 09-08-2012 |
| | | | WO | 2011159299 A1 | 22-12-2011 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61799129 A
- US 7404403 B
- US 6082363 E
- US 7210483 B
- US 7305990 B
- US 6830051 B